# EUROPÄISCHE PATENTSCHRIFT

(11) **EP 1 072 198 B1**
(45) Veröffentlichungstag und Bekanntmachung des Hinweises auf die Patenterteilung: **14.05.2008**
(21) Anmeldenummer: 99810681.9
(22) Anmeldetag: 28.07.1999
(51) Int. Cl.: A23L 1/304, A23L 1/302, A23L 1/30, A61K 33/26, A61K 31/20, A61K 31/355

(54) **Präparat zur Verwendung als Medikament und/oder Nahrungsmittelergänzung**
Preparation for use as medicament and/or nutritional supplement
Composition pour l'utilisation comme médicament et/ou complément alimentaire

(43) Veröffentlichungstag der Anmeldung: 31.01.2001
(73) Patentinhaber: Swiss Caps Rechte und Lizenzen AG, 9533 Kirchberg (CH)
(72) Erfinder: Engel, Dieter Wolfgang, 9524 Zuzwil (CH); Kokkinis, Georg, Dr., 9306 Freidorf (CH)
(74) Vertreter: Müller, Christoph Emanuel

(56) Entgegenhaltungen:
- EP-A- 0 094 149
- EP-A- 0 107 458
- DE-A- 2 639 177
- GB-A- 2 254 556
- US-A- 3 392 177
- PATENT ABSTRACTS OF JAPAN vol. 015, no. 221 (C-0838), 6. Juni 1991 (1991-06-06) & JP 03 067571 A (KANSAI PAINT CO LTD), 22. März 1991 (1991-03-22)
- Forth W , Henschler D. Pharmakologie und Toxikologie, 6, völlig neu bearbeitete Auflage, 1996, Seite 462
- Eisenbrand G, Schreier P. Römpp Lexikon Lebensmittelchemie, 1995,S345
- Aldrich Katalog, 1996, Seite 721 und 820
- Index Nominum, 1995, Seite 513 and 514
- Martindale, The Extra Pharmacopoeia,1989,Seite 1189-1192
- Prof. Dr. Falbe J. Prof. Dr. Regotz M. Römpp Chemie Lexikon,1992, Seite 3602
- Richtlinie 2002/46/EG des Europäischen Parlaments und des Rates vom 10 Juni 2002
- Förster H. Lacko L. Psysiologische Chemie, Seite 303
- Mutschler, Arzneimittelwirkungen, 5, Auflage 1986, Seite 385
- Meyers Taschenlexikon in 10 Bänden, Band 7, 1985
- Ibrahim et al, American Society for Nutricional Sciences, 1997, Seite 1401-1406
- Gallaher et al, 2004 IFT Annual Meeting, Las Vegas, Session 46, Dairy Foods : General(Abstract 46-2)
- Nishike et al , Bioscience, biotechnology and biochemistry, 1997, Vol 61, Nr.12

## Beschreibung

Die Erfindung betrifft ein Präparat gemäss Anspruch 1, zur Verwendung als Medikament und/oder Nahrungsmittelergänzung.

Der menschliche Körper ist auf die Zufuhr einer Reihe von essentiellen Substanzen angewiesen, die er nicht in der Lage ist selber zu synthetisieren. Das Risiko einer Unterversorgung ist bei ungenügender und/oder einseitiger Nahrungsmittelaufnahme dieser Substanzen gegeben. Trotz ausreichender und ausgewogener Nahrungsmittelzufuhr kann es unter besonderen Stresssituationen ebenfalls zu einer Unterversorgung an bestimmten Substanzen kommen. Stresssituationen treten in erster Linie bei besonderer körperlicher Belastung, wie zum Beispiel bei Leistungssport aber auch bei Schwangerschaft und Stillzeit ein.

Während Schwangerschaft und Stillzeit sind darüberhinaus eine Reihe von Substanzen für die gesunde Entwicklung des Kindes und für die Gesundheit der Mutter notwendig. Als eine der wichtigsten Substanzen aus der Klasse der Vitamine hat sich dabei die Folsäure erwiesen. Wie medizinische Studien zeigen, bestehen eindeutige Zusammenhänge zwischen Folsäure-Mangelerscheinungen und der gefürchteten Neuralrohrdefekte bei Neugeborenen, wie z.B. publiziert von R.D. Williams, FDA Consumer, May 1994, Seite 11-12 und O. Tönz, Schweizer Apothekerzeitung, 1996,17, Seite 424 - 426.

Untersuchungen jüngeren Datums geben Anlass zu der Schlussfolgerung, dass auf eine ausreichende Zufuhr an mehrfach ungesättigten Fettsäuren in freier Form und/oder als Glycerid während der Schwangerschaft und Stillzeit ebenfalls zu achten ist.

Zu den Polyen-Fettsäuren zählen drei unterschiedliche Familien, die sich durch die Stellung der ersten Doppelbindung, gerechnet vom Methylende der Fettsäuren her, unterscheiden. Als physiologisch wichtig haben sich die
- ω-3-mehrfach ungesättigten Fettsäuren (Linolensäure-Familie);
- ω-6-mehrfach ungesättigten Fettsäuren (Linolsäure-Familie) und
- ω-9-Fettsäuren
in medizinischen Studien erwiesen.

Die mehrfach ungesättigten Fettsäuren des ω-3- und ω-6-Typs bilden die Vorstufen für die Bildung wichtiger Gewebshormone wie z.B. Prostaglandine oder Leukotrine. Mehrere unabhängige Untersuchungen legen den Schluss nahe, dass eine höhere Zufuhr an Polyen-Fettsäuren aus der ω-6- und ω-3-Reihe die Sehschärfe des Neugeborenen verbessert und generell die frühkindliche Hirnentwicklung fördert. Eine besondere Rolle scheinen dabei Arachidonsäure (5,8,11,14-Eicosatetraensäure) und Docosahexaensäure, als Metaboliten der Linol- und Linolensäure zu spielen (D. Kunze, 49. DGF-Jahrestagung am 1.9.93, Karlsruhe).

Der weibliche Körper ist durch die Schwangerschaft einem erhöhten oxidativen Stress ausgesetzt. Als oxidativer Stress wird eine erhöhte Aktivität freier Radikale bezeichnet. Diese kann entweder durch eine höhere Produktion freier Radikale erzeugt werden oder durch eine Unterversorgung an schützenden antioxidativ wirkenden Substanzen. Der erhöhte oxidative Stress *in vivo* wird unter anderem für den schwangerschafts-induzierten Bluthochdruck verantwortlich gemacht, wie ausgeführt in S.J. Wisdom, R. Wilson, J.H. McKillop, J.J. Walker, Am. J. Obstet. Gynecol., 12/1991, Seite 1701 - 1704. Insbesondere in der Schwangerschaft besteht daher ein erhöhter Bedarf an antioxidativ wirkenden Substanzen.

Unter dem Begriff Antioxidantien werden organische Verbindungen verstanden, die unerwünschte, durch Sauerstoffeinwirkungen und andere oxidative Prozesse bedingte Veränderungen in anderen Substanzen hemmen oder verhindern. Tocopherole, Carotinoide, Tocotrienole, Ascorbinsäure, Selen, Polyphenole, Flavonoide, Flavonole, Flavone sind bekannt als Radikal-fänger oder sind eng an der Atmungskette beteiligt und schützen so die funktionelle und strukturelle Unversehrtheit von Lipiden und anderen wichtigen Stoffen wie Blut, Biomembranen und Zellinhalten.

Unter den Mineralien/Spurenelementen kommt Eisen eine spezielle Bedeutung zu. Der Eisenbedarf von Mutter und Kind ist ein bedeutender Faktor der prä- und postnatalen Gesundheit durch den Aufbau von speziellem fötalem Hämoglobin, dessen Umbau nach der Geburt und der Füllung des Eisenspeichers in Niere und Leber.

Zweiwertige Eisensalze gelten - in oraler Verabreichungsform - als die wirksamsten Eisenzufuhrstoffe. Das Mass der Eisenaufnahme ist jedoch oft relativ gering. Um eine ausreichende Bioverfügbarkeit zu gewährleisten, ist die Verabreichung hoher Mengen an Eisensalzen oftmals notwendig. Hohe Mengen an Eisensalzen und insbesondere die bei Dissoziation entstehende freien Eisenionen führen zu Unverträglichkeitsreaktionen, wie Übelkeit, Erbrechen und Durchfall bzw. Verstopfungen. Dreiwertige Eisensalze als Eisenlieferanten sind praktisch unwirksam, da sie im Darm zu Eisenoxid bzw. Eisenhydroxiden ausfallen.

Ein weiteres Problem, das mit der Verabreichung von Fe(II) verbunden ist, sind Lipid-Peroxidationsprozesse ungesättigter Fettsäuren, die neben anderen Ursachen auch unter der katalytischen Wirkung von Eisen (II) verlaufen. Durch die Lipid-Peroxidation entstehen mutagene und karzinogene Stoffe wie z.B. α-, β-ungesättigte Aldehyde. Aber auch Schäden an den Zellmembranen und anderen Zellkomponenten können auf diese Oxidationsprozesse zurückgeführt werden.

Antioxidantien wirken als Radikal-Fänger bei Lipid-Peroxidationsprozessen und können auf diese Weise die Kettenreaktion beenden. Auf der anderen Seite werden die als Radikal-Fänger eingesetzten Substanzen bei der "Neutralisation" selber verändert. Dies ist in den Fällen, in denen Vitamine als essentielle Substanzen dem Körper zugeführt werden sollen, nicht wünschenswert.

Autooxidation ungesättigter Fettsäuren in Gegenwart von Sauerstoff und Katalysatoren spielt eine entscheidende und in ihren Effekten nicht erwünschte Rolle beim Ranzigwerden von Fetten bzw. Ölen (S. Ullmann's Encyclopedia of Industrial Chemistry, Eds. L. Kandy, J.F. Rounsville, G. Schulz, 5^{th} ed., Vol. A 10, S. 190 ff., 1987). Dieser Prozess wird durch das Vorhandensein von Metall-Katalysatoren gefördert bzw. beschleunigt.

Insbesondere die durch die Autooxidation entstehenden freien Fettsäuren und volatilen Carbonylverbindungen, wie z.B. Aldehyde und Ketoverbindungen sind für den unangenehmen Beigeschmack und Geruch gealterten Fettes verantwortlich.

Die bei der Autooxidation gebildeten Primärprodukte, die Hydroperoxide, die Säuerzahl und der Anteil volatiler Carbonylverbindungen, insbesondere die Aldehyde, dienen als Messparameter und für die Festlegung von Grenzwerten für die Lagerung und Herstellung.

Die Formulierung eines Präparates, welches eine Kombination mehrfach ungesättigter Fettsäuren in freier oder chemisch gebundener Form und physiologisch wirksamer Eisensalze, insbesondere Eisen (II) Salze, enthält, ist aus Gründen der Reaktivität der Substanzen und der daraus resultierenden Instabilität solcher Präparate mit grossen Schwierigkeiten behaftet und wurde bis anhin nicht realisiert.

Aufgabe der vorliegenden Erfindung ist die Vermeidung der Nachteile des Standes der Technik.

Es ist weiterhin Aufgabe der Erfindung, physiologisch wirksame eisenhaltige Verbindungen, mit ungesättigten Fettsäuren in freier und/oder chemisch gebundener Form in einem Präparat zu kombinieren, ohne dass es zu einer wesentlichen Beeinträchtigung der physiologischen Wirksamkeit einer oder beider Komponenten kommt. Die Kombination soll ohne vorangehende aufwendige Schutzschritte, wie z.B. einer Mikroverkapselung einzelner oder aller Komponenten möglich sein. Eine weitere Aufgabe besteht in der Bereitstellung eines lagerstabilen Multikomponenten-Präparates, welches neben physiologisch wirksamen eisenhaltigen Verbindungen und ungesättigten Fettsäuren in freier und/oder chemisch gebundener Form noch weitere physiologisch wirksame Komponenten enthält, und insbesondere zur Herstellung eines Medikamentes und/oder einer Nahrungsmittelergänzung zur Prävention und Behandlung von Mangelerscheinungen eingesetzt wird.

Diese Aufgaben werden gelöst durch die kennzeichnenden Merkmale des Anspruchs 1.

Insbesondere werden sie gelöst durch ein Präparat, welches mindestens einen physiologisch wirksamen Eisen-Komplex und mindestens eine ungesättigte Fettsäure in freier und/oder chemisch gebundener Form enthält.

Unter "physiologisch wirksam" soll verstanden werden, dass der Eisen-Komplex zum einen keine bzw. nur unwesentliche unerwünschte Nebenwirkungen im menschlichen Körper entfaltet und zum anderen in der Lage ist Eisenmangel vorzubeugen bzw. zu behandeln und zu beheben.

Das erfindungsgemässe Präparat wird als Medikament und/oder als Nahrungsmittelergänzung eingesetzt.

Im weitesten Sinne wird unter dem Begriff "Komplex" eine Verbindung höherer Ordnung verstanden, die formal durch stöchiometrischen Zusammenschluss von selbstständig und unabhängig voneinander existensfähigen Molekülen und/oder Ionen entstehen. Dies ist als Gegensatz zu den Verbindungen erster Ordnung aufzufassen, an deren Entstehung Atome beteiligt sind.
Bei der Bildung von Komplexen werden an ein ungeladenes oder geladenes Atom Z, dem Zentralatom bzw. -ion, entsprechend seiner Koordinationszahl n mehrere ungeladene oder geladene, ein oder mehratomige Gruppen L (Liganden) angelagert. Eisen, als Übergangsmetall, bindet solche Liganden, die über freie oder π-Elektronenpaare verfügen, und welche mit den unbesetzten Hybridorbitalen des Eisens zur Bildung eines Komplexes in Wechselwirkung treten können.

In einer bevorzugten Ausführungsform sind die Liganden mindestens zweizähnig, d.h. der bzw. die Ligand(en) können sich mit mindestens zwei komplexbildenden Atomen an ein Zentralatom anlagern und sogenannte Chelatverbindungen bilden. Die komplexbildenden Atome sind dabei in der Regel O,S und N. Komplexe mit zwei- und mehrzähnigen Chelat-Liganden sind in der Regel beständiger als Komplexe mit nichtverbrückten Liganden.

Da die physiologisch wirksamen Eisen-Komplexe sich innerhalb des Körpers in Konkurrenz zu anderen Komplexbildnern, wie sie z.B. durch die Nahrung bereitgestellt werden (Proteine, Peptide, Polycarbonsäure, Zucker, Phosphate, Thiole und andere potentielle Liganden wie sie im Darm-Lumen vorkommen) befinden, muss die Komplexstabilität so gross sein, dass der eingesetzte Eisenkomplex Konkurrenzreaktionen mit den "Konkurrenz"-Komplexbildern im wesentlichen nicht unterliegt. Die Stabilität muss jedoch geringer sein als die des mukosalen Transferrins, damit das Eisen an den Akzeptorstellen des Transfersystems übernommen werden kann. Die Komplexstabilität muss bevorzugt so gross sein, dass das Auftreten freier Eisenionen in Wasser und vorzugsweise auch unter pH-Bedingungen wie sie im Magen- und Darmtrakt vorherrschen im wesentlichen unterbleibt. Die im erfindungsgemässen Präparat eingesetzten Eisenkomplexe können jedoch eine gewisse Wasserlöslichkeit aufweisen.

Eisenkomplexe, die oben genannte Bedingungen erfüllen und zur Behandlung von Eisenmangel eingesetzt werden, sind bekannt. Es hat sich nun überraschenderweise gezeigt, dass mindestens einer dieser Eisenkomplexe und mindestens eine mehrfach ungesättigte Fettsäure in freier oder chemisch gebundener Form in einem Präparat kombiniert werden können, ohne dass es unter Standardbedingungen innerhalb von 6 Monaten zu wesentlichen Veränderungen, z.B. durch Oxidations- und Hydrolyseprozesse der ungesättigten Fettsäuren kommt.

Das erfindungsgemässe Präparat sollte Peroxidzahlen (POZ) von 10 (meq 0₂/kg) nicht überschreiten, bevorzugt liegt POZ bei einem Höchstwert von 5 meq 0₂/kg und noch bevorzugter bei Höchstwerten von 3 meq 0₂/kg (Methode 7/5.2 nach Schweizer Lebensmittelbuch, Juli 1994). Weiterhin dient die Bestimmung der Aldehyde und freien Fettsäuren als Messparameter.

Als Standardbedingungen werden eine Temperatur von 25°C ± 2°C eine relative Luftfeuchtigkeit von 60% ± 5%und ein Druck von 1 bar definiert.

Einer bevorzugten Ausführungsform der vorliegenden Erfindung entspricht die Oxidationsstufe +III des Eisens im Eisenkomplex. Eine weitere bevorzugte Ausführungsform ist das Eisen in der Oxidationsstufe ±0 im Eisenkomplex.

Die Verwendung von Komplexen, die Eisen in der Oxidationsstufe +II enthalten und den genannten Bedingungen entsprechen können im erfindungsgemässen Präparat entweder alleine oder in Kombination ebenfalls Anwendung finden.

Für das Präparat der vorliegenden Erfindung eignen sich die in der Literatur beschriebenen physiologisch wirksamen Eisen(III)-Komplexe unter der Vorraussetzung sie erfüllen die bereits genannten Bedingungen.

Bevorzugt werden die in EP 107 458 beschriebenen 3-hydroxy-4-pyron Eisenkomplexe und Derivate hiervon eingesetzt. Die Derivate umfassen solche 3-hydroxy-4-pyron Eisenkomplexe, bei dem eines oder mehrere der Wasserstoffatome des Pyronringes durch eine aliphatische Kohlenstoffkette ersetzt ist. Bevorzugt enthält die Kohlenstoffkette eins bis sechs Kohlenstoffatome. In einer bevorzugten Ausführungsform ist das Derivat des 3-hydroxy-4-pyrons Maltol (3-hydroxy-2-methyl-4-pyron).

Anwendung im erfindungsgemässen Präparat finden weiterhin die in EP 0 094 149 beschriebenen 3-hydroxypyrid-2-on Eisenkomplexe und Derivate hiervon.

Als erfindungsgemäss geeignet erwiesen haben sich darüberhinaus Eisen(III)-hydroxidpolymaltosekomplexe, wie sie in der Deutschen Apotheker Zeitung 121. Jahrgang, Nr. 41, Seite 2193 bis 2195 von H. Jakobs beschrieben werden.

Als Eisenkomplexe, welche Eisen in der Oxidationsstufe 0 aufweisen, haben sich in erster Linie Eisenpentacarbonylkomplexe (Eisen-Ferronyl gemäss Food Chemical Codex, 4^{th} ed.,1999) und Ferrocen als bevorzugte Ausführungsformen gezeigt.

Die bevorzugten Fe(III) 3-hydroxy-4-pyron-Komplexe, sowie die Fe(III) 3-hydroxy-4-pyrid-2-on-Komplexe und ihre Derivate, insbesondere Maltol, können in der neutralen Form und damit in einem Verhältnis Ligand : Fe (+III) von 3:1 vorliegen. Aber auch Eisen(III)-Komplexe, die molare Verhältnisse des Liganden : Eisen von 2:1 und 1:1 aufweisen, können erfindungsgemäss eingesetzt werden und sollen im Zuge der vorliegenden Erfindung unter die Bezeichnung der neutralen Form fallen. Diese Verhältnisse machen jedoch das Vorhandensein weiterer Anionen wie z.B. Chlorid erforderlich, um die Ladung des Zentralatoms auszugleichen.

Der Fe(III)-Gehalt kann je nach Komplex aber auch innerhalb der gleichen Art von Komplex variieren. Als tägliche Eisenzufuhr eines Erwachsenen wird im allgemeinen eine Menge zwischen 2 bis 4 mg Eisen als ausreichend angesehen. Die Menge des in dem erfindungsgemässen Präparat eingesetzten Eisenkomplexes sollte demzufolge so vorliegen, dass Eisen in einem Bereich zwischen 0,1 bis 20 mg, bevorzugt in einem Bereich von 5 bis 18 mg und noch bevorzugter in einem Bereich von 10 bis 15 mg im Präparat vorhanden ist, um Eisenmangel zu beheben bzw. vorzubeugen. Diese Werte beziehen sich auf eine einmalige tägliche Einnahme einer entsprechenden Darreichungsform.

Neben den Eisen(III)-hydroxypyron-, Eisen(III)-hydroxypyridon- und Eisen(III)-hydroxidpolymaltosekomplexen, die entweder alleine oder als Mischungen miteinander Verwendung finden, können weitere Fe(III)-Komplexe, wie z.B. Eisen(III)-citratkomplexe ebenfalls entweder alleine oder in Mischungen mit den vorgenannten Komplexen im erfindungsgemässen Präparat eingesetzt werden.

Ein weiterer Faktor, der eine Rolle bei der Bioverfügbarkeit des Eisens spielt, ist die Fähigkeit der Eisenkomplexe biologische Membranen zu durchdringen. Eine der möglichen Indikatoren für diese Membranpermeation ist Kₚₐᵣₜ. Kₚₐᵣₜ bezeichnet das Verhältnis zwischen der Konzentration der Verbindung in n-Octanol zu der Konzentration der Verbindung in der wässrigen Phase. Obwohl sowohl der Eisenkomplex als auch seine korrespondierende metallfreie Verbindung zum Durchtritt durch die Biomembranen befähigt sein sollten, sollten beide auch ein gewisses Ausmass an Wasserlöslichkeit aufweisen. Im Zuge der vorliegenden Erfindung weisen die Eisenkomplexe bevorzugt Kₚₐᵣₜ- Werte in einem Bereich zwischen 0.02 und 6.0, bevorzugt zwischen 0.1 und 4.0 und noch bevorzugter zwischen 0.2 und 1.0 auf. Der metallfreie Komplex besitzt bevorzugt Kₚₐᵣₜ- Werte zwischen 0.05 und 3.0, bevorzugt zwischen 0.1 und 2.0 und noch bevorzugter zwischen 0.2 und 1.0.

In einer bevorzugten Ausführungsform sind die in dem erfindungsgemässen Präparat eingesetzten ungesättigten Fettsäuren in freier oder chemisch gebundenen Form ausgewählt aus der Gruppe bestehend aus ω-3 und ω-6-mehrfach ungesättigten Fettsäuren.

Der Begriff "chemisch gebunden" soll für alle die ungesättigten Fettsäuren stehen, die in einer anderen als der freien Form vorliegen, z.B. in Form ihrer Glyceride (Mono-, Di-oder Triglyceride) in Ölen oder Fetten bzw. anderweitiger Ester. Sie werden sowohl alleine oder in Mischungen miteinander verwendet.

Bevorzugt werden sie in der Form, in der sie bei Gewinnung anfallen, mit den übrigen Komponenten im erfindungsgemässen Präparat kombiniert, so z.B. Docosahexaensäure und Eicosapentaensäure in Konzentration und Struktur wie sie z.B. in Fischölen vorliegen. Sie können jedoch auch gereinigt, hoch angereichert und bei Bedarf umgeestert als Glyceride oder Fettsäuren kombiniert werden.

Bevorzugt gehören die ω-3-mehrfach ungesättigten Fettsäuren zur Linolensäure-Familie, während die ω-6-mehrfach ungesättigten Fettsäuren bevorzugt aus der Linolsäure-Familie stammen.

Die ω-3 und ω-6-mehrfach ungesättigten Fettsäuren werden bevorzugt in einer Menge von 5 bis 75 Gew.% bezogen auf das Gesamtgewicht des Präparates, bevorzugter in einer Menge von 30 bis 70 Gew.% und noch bevorzugter in einer Menge von 50 bis 60 Gew.% eingesetzt. Diese Mengen können von einer der Fettsäuren alleine oder von Mischungen beider erhalten werden.

Für die meisten der im erfindungsgemässen Präparat anwendbaren Substanzen wird eine Mengenangabe bevorzugt, die sich nach den RDA (recommended daily allowance)-Werten richtet. Die Menge Säuren aus der Linolen- und Linolsäure-Familie, insbesondere an ω-3 und ω-6 Fettsäuren, liegt zwischen 30mg und 1000mg (100% freie Fettsäure) pro Darreichungsform bei einmaliger täglicher Zufuhr, bevorzugt zwischen 50mg und 800mg und noch bevorzugter zwischen 80 und 250mg.

In einer bevorzugten Ausführungsform ist die ω-3 ungesättigte Fettsäure Docosahexaensäure (C-22:ω-3) und/oder Eicosapentaensäure und/oder Eicosatetraensäure. Besonders in Fischölen Docosahexaensäure und Eicosapentaensäure kommt als Mischung insbesondere in Fischöelen vor.

In einer weiteren bevorzugten Ausführungsform sind die ungesättigten Fettsäuren der Linolen- und Linolsäurefamilie pflanzlichen Ursprungs und enthalten in fetten Ölen, wie sie z.B. aus Borretsch oder Nachtkerzen gewonnen werden.

Besonders vorteilhaft werden im erfindungsgemässen Präparat fett- und wasserlösliche Radikal-fänger aus der Gruppe der Tocopherole, Carotinoide, Tocotrienole, Polyphenole, Flavonoide Flavonole, Flavone und Ascorbat, sowie zusätzliche wasser- und/oder fettlösliche Vitamine eingesetzt.

Als Antioxidants aus der Gruppe der wasserlöslichen Substanzen findet bevorzugt Ascorbinsäure, Flavonoide und Falvone im erfindungsgemässen Präparat Einsatz. Aus Stabilitätsgründen kann Ascorbinsäure jedoch nicht in einer Kombination mit Eisenferronyl eingesetzt werden.

Als Antioxidants aus der Gruppe der fettlöslichen Substanzen findet bevorzugt die Carotinoide (Carotin, Lutein, Lycopin, Zeaxanthin, etc.) und Tocotrienole Anwendung. Fettlösliche Vitamine mit einer Radikalfängerfunktion sind insbesondere bevorzugt Tocoperol aus der Vitamin E-Gruppe, zu der auch die Ester von α, β, γ, δ-Tocopherolen zählen.

Bei einmaliger täglicher Verabreichung des erfindungsgemässen Präparates sollte die Darreichungsform für Erwachsene eine Menge an Carotinoiden (>60% β-Form) und Polyhydroxyphenolen wie Flavonoiden, Flavonen, etc.) zwischen 4 und 25mg, bevorzugt von 6 bis 15mg beinhalten. Die Mengen an Vitaminen richtet sich nach den RDA-Werten (Erwachsene 25 bis 50Jahre) und liegt für eine einmalige tägliche Zufuhr pro Darreichungsform für Erwachsene für Vitamin C zwischen 20mg und 100mg, für Vitamin E zwischen 10 und 25mg.

In einer weiteren bevorzugten Ausführungsform enthält das erfindungsgemässe Präparat zusätzlich Folsäure in einer Menge pro Darreichungsform bei einmaliger täglicher Zufuhr in einem Bereich von 0.1g bis 0.6g (87.8% Reinheit) bevorzugt in einer Menge von 0.2g bis 0.5g und noch bevorzugter von 0.25 bis 0.4g.

Folsäure erleichtert die Behandlung von Anemie, welche auf Eisenmangel zurückzuführen ist und wird darüberhinaus, wie bereits beschrieben, zur Prävention von Neuronalrohrdefekten eingesetzt.

Des weiteren kann auch eine Zinkquelle in einer Menge pro Darreichungsform (einmalige tägliche Zufuhr) von 0.5 bis 20mg, bevorzugt von 5 bis 17mg und noch bevorzugter von 12 bis 15mg dem erfindungsgemässen Präparat zugesetzt werden.

Insbesondere kann das erfindungsgemässen Präparates verwendet werden zur Herstellung eines Medikamentes und/oder Nahrungsmittelergänzung zur Behandlung und Prävention von Mangelerscheinungen.

Besonders eine Kombination von Folsäure, Antioxidantien, insbesondere antioxidativ wirkender Vitamine, physiologisch wirksamer Eisen-Komplexen und ungesättigter Fettsäuren in freier und oder chemisch gebundener Form , insbesondere mit Fettsäuren des ω-3- und/oder ω-6-Typs hat sich als vorteilhaft in der der Schwangerschaft und Stillzeit erwiesen. Insbesondere wird durch den Eisenkomplex zwar eine Eisenzufuhr gewährleistet, unerwünschte neben- bzw. katalytische Wirkungen des Eisens jedoch verhindert. Dies ist in erster Linie auf die geringe Konzentration freier Eisenionen zurückzuführen, da die aus dem Komplex freigesetzte Eisenmengen unmittelbar vom Körper aufgenommen werden. Die Konzentration freier Eisenionen liegt somit sowohl im Präparat als auch im Körper unterhalb der Menge, welche unerwünschte Veränderungen körperlicher oder anderer Verbindungen in grossem Ausmass verursacht.

Das erfindungsgemässe Präparat kann in Darreichungsformen verabreicht werden, die das Präparat in einer Menge enthalten, die für eine einmalige tägliche Zufuhr ausreichend ist. Die erforderliche tägliche Menge der physiologisch wirkungsvollen Substanzen im erfindungsgemässen Präparat können jedoch auch verteilt auf zwei oder mehrere Darreichungsformen über den Tag verteilt werden. Als Darreichungsform geeignet sind Kapseln (Hartkapseln (banderolliert und gesiegelt) und Weichkapseln), Trinklösungen und Sirup angeboten werden. Als besonders geeignet hat sich die Verkapselung in, z.B. Weichgelatin-, Hartgelatin- und Stärkekapseln erwiesen.

Die Verkapselung geschieht im geschlossenen System unter Stickstoff unter weitgehend wasserfreien Bedingungen nach starker Trocknung der einzelnen Inhaltsstoffe.

Abhängig von der Darreichungsform finden unterschiedliche Hilfssubstanzen und Trägermaterialien Einsatz. Eingesetzt wird z.B. Bienewachs als Wasserschutz und zur Viskositätserhöhung, Mono-und Di-Glyceride von Speisefettsäuren als Emulgatoren, Sorbitol/Manitol als nicht diffundierende Weichmacher (wenn in der Darreichungsform Kapsel), Aromen zur Geruchs- und Geschmacksinertisierung von z.B. Fischöl.

Weichgelatinkapseln, welche das erfindungsgemässe Präparat einkapseln, werden bevorzugt im Rotary-Die-Prozess hergestellt.

Die Darreichungsformen , insbesondere in der Ausführungsform als Kapsel können magensaftresistente Lackierungen enthalten, um gegebenenfalls je nach Zusammensetzung des Präparates eine verzögerte Freigabe (im Darm) zu erreichen. Auch die Vernetzung der Gelatine in der Hülle kann je nach Zusammensetzung des Präparates bestimmte Retardwirkungen verursachen.

Die vorliegende Erfindung wird weiterhin anhand von Beispielen erläutert.

### Bestimmung des Kₚₐᵣₜ-Wertes:

Bestimmt wird die Verteilung jeweils für den Eisenkomplex als auch für die entsprechende metallfreie Verbindung zwischen n-Octanol und wässrigem Tris Hydrochloride (20mM, pH 7,4; Tris: 2-amino-2-hydroxymethylpropan-1,3-diol). Die Konzentration der Substanzen in den jeweiligen Phasen wird bei 20°C mittels Spektrophotometrie gemessen. 5 ml einer 10⁻⁴M wässrigen Lösung aus Eisenkomplex bzw. metallfreier Verbindung werden mit 5 ml n-Octanol eine Minute gemischt. Die dabei entstehende wässrige n-Octanol-Mischung wird bei 1,000G 30 Sekunden zentrifugiert. Die zwei resultierenden Phasen werden für eine Konzentrationsbestimmung voneinander abgetrennt. Die metallfreie Verbindung wird in einem Bereich von 220 bis 340 Nanometer gemessen, während für die Konzentrationsbestimmung des korrespondierenden Eisenkomplexes ein Bereich zwischen 340 und 640 Nanometer ausgewählt wird. Diese Bereiche gelten auch für den 3:1 Maltol-Eisen(III)-Komplex.

### Nachfolgende Tabelle zeigt Kₚₐᵣₜ-Werte einiger ausgewählter Komplexe

| Verbindung | Verteilungskoeffizient Kₚₐᵣₜ | |
|---|---|---|
| | Metallfreie Verbindung | Eisen-Komplex [Fe^{III}-(Verbindung)₃] |
| 3-hydroxy-1-methylpyrid-2-on | 0,44 | 0,10 |
| 1-ethyl-3-hydroxypyrid-2-one | 0,52 | 1,06 |
| 3-hydroxy-2-methyl-1-propylpyrid-4-on | 0,67 | 0,53 |
| 3-hydroxy-2-methyl-4-pyrome | 0,66 | 0,5 |
| Fe^{III}-EDTA | 0,001 | 0,0015 |

### Beispiel 1:

### Schwangerschaftspräparat

| | |
|---|---|
| Fischöl (Triglycerid Form) mit 10% Eicosapentaensäure(EPA) und mind. 33% Docosahexaensäure (DHA) (entsprechend >50 mg EPA, >150 mg DHA pro Dosis) | 500 mg |
| Eisen (III)-Hydroxid Polymaltose Komplex mit >28% Fe, (entsprechend 18 mg Fe/Dosis) | 65 mg |
| Folsäure Ph.Eur (chem. reine Folsäure >87.8%) | 0.57 mg |
| D-α-Tocopherol Konzentrate aus Soyaöl Fraktionen korr. 36 IU in Form von 2R,4'R,8'R-α-Tocopherol (24.1 mg) | 37.8 mg |
| Mono-Di-glyceride der Stearinsäure | 50.0 mg |
| In einer Weichgelatinehülle bestehend aus Gelatine, Glycerin, Carob, Wasser | |

### Beispiel 2: (Geeignet während der letzten Phase der Schwangerschaft und der Stillzeit)

| | |
|---|---|
| Borretschöl (fettes Oel aus semen Borago officinalis) mit 18-25% Gamma-Linolensäure (C18:3 ω6) (corr. >90 mg GLA) | 500 mg |
| D-α-Tocopherol Konzentrate aus Soyaöl Fraktionen korr. 12 IU in Form von 2R,4'R,8'R-α -Tocopherol (8.05 mg) | 12,6 mg |
| Eisen Ferronyl (FCC¹) corr. 15 mg Fe (=approx. 1RDA²) | 15.03 mg |
| All-trans-β-Carotin, 30% Suspension in Pflanzenöl, corr. Beta-Carotin 6mg | 21.0 mg |
| Folsäure Ph.Eur (chem. reine Folsäure >87.8%) (0.4 g) | 0,57 mg |
| Mono-Di-Glyceride der Stearin- und Oelsäure | 50,0 mg |
| In einer Weichgelatinehülle bestehend aus Gelatine, Glycerin, Sorbitol, Titandioxid, Eisenoxiden, Wasser | |

| | |
|---|---|
| ¹ FCC = Food chemical Codex 4^{th} ed. 1999 ² RDA = Recommended daily intake | |

### Beispiel 3:

| | |
|---|---|
| Fischöl (Triglycerid-Form) mit 18% Eicosapentaensäure und mind. 12% Docosahexaensäure (entsprechend >63 mg Eicosapentaensäure, 42 mg Docosahexaensäure pro Dosis) | 350 mg |
| Nachtkerzenöl (fettes Öl aus semen Oenothera biennis) mit 10% Gamma-Linolensäure (C18:3 omega6) | 500 mg |
| Folsäure Ph.Eur (chem. reine Folsäure > 87.8%) (0.2 g) | 0.29 mg |
| Eisen (III) polysaccharat Ph.Helv. VII³ (2.8-3.0% Fe) Corr. 5 mg Fe(III) | 167.0 mg |
| DL-α-Tocopherylacetat Ph.Eur. (corr. 10 IU) | 10.5 mg |
| 30% Carotin Konzentrat aus Palmfrüchten (Biocon, Fa. Quest) (davon ca. 57% β-Carotin, 32% α-Carotin, 11% γ-carotin, lycopin etc. Verhältnis cis/trans 40/60 | 10.0 mg |
| Ascorbinsäure Ph.Eur. (corr. 30 mg) | 33.00 mg |
| Pflanzliche Fette z.T. Hydriert | 18.0 mg |
| Bienenwachs gelb Ph.Eur. | 24.0 mg |
| In einer Weichgelatinehülle bestehend aus Gelatine, Glycerin, Sorbitol, Titandioxid, Eisenoxiden, Wasser | |

| | |
|---|---|
| ³ Ph.Helv. VII = Pharmacopoeia Helvetica 7^{th} edition | |

## Patentansprüche

1. Präparat enthaltend mindestens einen physiologisch wirksamen Eisen-Komplex und mindestens eine ungesättigte Fettsäure in freier und/oder chemisch gebundener Form, mit der Massgabe dass es sich nicht um ein Gemisch aus Eisenpentacarbonyl und/oder einem Komplex aus Eisentricarbonyl und einem mehrfach ungesättigten pflanzlichen Öl aus der Gruppe aus Leinsamenöl, Sojabohnenöl oder Safloröl mit einem mehrfach ungesättigten pflanzlichen Öl aus der Gruppe aus Leinsamenöl, Sojabohnenöl oder Safloröl handelt,
**dadurch gekennzeichnet, dass** das Präparat ein Arzneimittel oder ein Nahrungsergänzungsmittel ist.

2. Präparat gemäss Anspruch 1, **dadurch gekennzeichnet, dass** das Eisen im Eisenkomplex in der Oxidationsstufe +III vorliegt.

3. Präparat gemäss Anspruch 1, **dadurch gekennzeichnet, dass** das Eisen im Eisenkomplex in der Oxidationsstufe 0 vorliegt.

4. Präparat gemäss einem der Ansprüche 1 bis 3, **dadurch gekennzeichnet, dass** der Eisenkomplex Kₚₐᵣₜ-Werte in einem Bereich von 0,02 bis 6,0 aufweist und die korrespondierende metallfreie Verbindung Kₚₐᵣₜ-Werte von 0,05 bis 3,0.

5. Präparat gemäss einem der Ansprüche 1 bis 4, **dadurch gekennzeichnet, dass** der Eisen-Komplex ausgewählt ist aus der Gruppe bestehend aus Eisenchelatkomplexen, Eisen-(III)-maltol, Eisen-(III)-hydroxidpolymaltose, Ferrocen und Eisenpentacarbonyl.

6. Präparat gemäss einem der Ansprüche 1 bis 5, **dadurch gekennzeichnet, dass** die ungesättigte Fettsäure in freier und/oder chemisch gebundener Form in einer Menge von 30mg bis 1000mg bezogen auf 100% freie reine Fettsäure pro Darreichungsform bei einmaliger täglicher Zufuhr vorliegt, bevorzugt in einer Menge von 50mg bis 800mg und noch bevorzugter in einer Menge von 80mg bis 250mg.

7. Präparat gemäss einem der Ansprüche 1 bis 6, **dadurch gekennzeichnet, dass** die mehrfach ungesättigte Fettsäure ausgewählt ist aus der Gruppe bestehend aus ω-3-Fettsäuren, insbesondere aus der Linolensäure-Familie und ω-6-Fettsäuren, insbesondere aus der Linolsäurefamilie.

8. Präparat gemäss einem der Ansprüche 1 bis 7, **dadurch gekennzeichnet, dass** die ungesättigte Fettsäure ausgewählt ist aus der Gruppe bestehend aus Eicosapentaensäure, 5,8,11,14 Eicosatetraensäure und Docosahexaensäure.

9. Präparat gemäss einem der Ansprüche 1 bis 8, **dadurch gekennzeichnet, dass** das Präparat mindestens ein Antioxidants enthält, insbesondere ausgewählt aus der Gruppe der wasser- und/oder fettlöslichen Vitamine.

10. Präparat gemäss Anspruch 9, **dadurch gekennzeichnet, dass** das fettlösliche Vitamin ausgewählt wird aus der Gruppe bestehend aus Tocopherolen, Carotinoiden, Tocotrienolen, Polyphenolen, Flavonoiden, Flavonolen und Flavonen, bevorzugt in einer Menge von 4mg bis 25mg pro Darreichungsform bei einmaliger täglicher Zufuhr.

11. Präparat gemäss Anspruch 10, **dadurch gekennzeichnet, dass** die Tocopherole ausgewählt sind aus der Vitamin E-Gruppe, bevorzugt in einer Menge von bis zu 25 mg pro Darreichungsform bei einmaliger täglicher Zufuhr.

12. Präparat gemäss Anspruch 11, **dadurch gekennzeichnet, dass** das wasserlösliche Vitamin Ascorbinsäure enthält, bevorzugt in einer Menge von 20mg bis 100mg pro Darreichungsform bei einmaliger täglicher Zufuhr.

13. Präparat gemäss einem der Ansprüche 1 bis 12, **dadurch gekennzeichnet, dass** das Präparat Folsäure enthält, bevorzugt in einer Menge von 0.1g bis 0.6g bei 87.8% Reinheit der Folsäure pro Darreichungsform bei einmaliger täglicher Zufuhr und noch bevorzugter in einer Menge von 0.2 bis 0.5g und noch bevorzugter 0.25 bis 0.4g.

14. Verwendung eines Präparates gemäss einem der Ansprüche 1 bis 13 für die Herstellung eines Medikamentes zur Vorbeugung und/oder Behandlung von Mangelerscheinungen, insbesondere während Schwangerschaft und Stillzeit.

15. Kapsel, insbesondere Weichgelatinekapsel, enthaltend ein Präparat gemäss einem der Ansprüche 1 bis 13.

## Claims

1. A preparation containing at least one physiologically active iron complex and at least one unsaturated fatty acid in free and/or chemically bound form, with the proviso that it is not a mixture of iron pentacarbonyl and/or a complex of iron tricarbonyl and a polyunsaturated vegetable oil from the group consisting of linseed oil, soya bean oil or safflower oil with a polyunsaturated vegetable oil from the group consisting of linseed oil, soya bean oil or safflower oil, **characterised in that** the preparation is a medication or a food supplement.

2. A preparation according to claim 1 **characterised in that** the iron is present in the iron complex in the oxidation stage +III.

3. A preparation according to claim 1 **characterised in that** the iron is present in the iron complex in the oxidation stage 0.

4. A preparation according to one of claims 1 to 3 **characterised in that** the iron complex has Kₚₐᵣₜ values in a range of from 0.02 to 6.0 and the corresponding metal-free compound has Kₚₐᵣₜ values of from 0.05 to 3.0.

5. A preparation according to one of claims 1 to 4 **characterised in that** the iron complex is selected from the group consisting of iron chelate complexes, iron (III) maltol, iron (III) hydroxide polymaltose, ferrocene and iron pentacarbonyl.

6. A preparation according to one of claims 1 to 5 **characterised in that** the unsaturated fatty acid in free and/or chemically bound form is present in an amount of from 30 mg to 1000 mg with respect to 100% free pure fatty acid per administration form with a single daily intake, preferably in an amount of from 50 mg to 800 mg and still more preferably in an amount of from 80 mg to 250 mg.

7. A preparation according to one of claims 1 to 6 **characterised in that** the polyunsaturated fatty acid is selected from the group consisting of ω-3-fatty acids, in particular from the linolenic acid family and ω-6-fatty acids, in particular from the linoleic acid family.

8. A preparation according to one of claims 1 to 7 **characterised in that** the unsaturated fatty acid is selected from the group consisting of eicosapentaenoic acid, 5,8,11,14 eicosatetraenoic acid and docosahexaenoic acid.

9. A preparation according to one of claims 1 to 8 **characterised in that** the preparation contains at least one antioxidant, in particular selected from the group of water-soluble and/or fat-soluble vitamins.

10. A preparation according to claim 9 **characterised in that** the fat-soluble vitamin is selected from the group consisting of tocopherols, carotinoids, tocotrienols, polyphenols, flavonoides, flavonols and flavones, preferably in an amount of from 4 mg to 25 mg per administration form with a single daily intake.

11. A preparation according to claim 10 **characterised in that** the tocopherols are selected from the vitamin E group, preferably in an amount of up to 25 mg per administration form with a single daily intake.

12. A preparation according to claim 11 **characterised in that** the water-soluble vitamin contains ascorbic acid, preferably in an amount of from 20 mg to 100 mg per administration form with a single daily intake.

13. A preparation according to one of claims 1 to 12 **characterised in that** the preparation contains folic acid, preferably in an amount of from 0.1 g to 0.6 g with 87.8% purity of the folic acid per administration form with a single daily intake and still more preferably in an amount of from 0.2 to 0.5 g and still more preferably from 0.25 to 0.4 g.

14. Use of a preparation according to one of claims 1 to 13 for the production of a medicament for preventing and/or treating deficiency symptoms, particularly during pregnancy and lactation.

15. A capsule, in particular a soft gelatine capsule, containing a preparation according to one of claims 1 to 13.

## Revendications

1. Préparation contenant au moins un complexe de fer physiologiquement actif et au moins un acide gras insaturé sous forme libre et/ou chimiquement liée, avec la réserve qu'il ne s'agisse pas d'un mélange de pentacarbonyle de fer et/ou d'un complexe de tricarbonyle de fer et d'une huile végétale poly-insaturée du groupe constitué de l'huile de lin, l'huile de soja et l'huile de carthame avec une huile végétale poly-insaturée du groupe constitué de l'huile de lin, l'huile de soja et l'huile de carthame, **caractérisée en ce qu'**elle est un médicament ou un complément alimentaire.

2. Préparation selon la revendication 1, **caractérisée en ce que** le fer dans le complexe de fer est au degré d'oxydation + III.

3. Préparation selon la revendication 1, **caractérisée en ce que** le fer dans le complexe de fer est au degré d'oxydation 0.

4. Préparation selon l'une des revendications 1 à 3, **caractérisée en ce que** le complexe de fer présente des valeurs Kpart dans la plage de 0,02 à 6,0 et le composé non métallique correspondant présente des valeurs Kpart de 0,05 à 3,0.

5. Préparation selon l'une des revendications 1 à 4, **caractérisée en ce que** le complexe de fer est choisi dans le groupe constitué des complexes chélateurs du fer, le fer (III)-maltol, le fer (III) - hydroxypolymaltose, le ferrocène et le pentacarbonyle de fer.

6. Préparation selon l'une des revendications 1 à 5, **caractérisée en ce que** l'acide gras insaturé se présente sous une forme libre et/ou chimiquement liée en une quantité de 30 mg à 1000 mg rapportés à 100 % d'acide gras pur libre par forme galénique en un apport quotidien unique, de préférence en une quantité de 50 mg à 800 mg et de manière encore plus préférée, en une quantité de 80 mg à 250 mg.

7. Préparation selon l'une des revendications 1 à 6, **caractérisée en ce que** l'acide gras poly-insaturé est choisi dans le groupe constitué des acides gras ω-3, en particulier de la famille des acides linoléniques et des acides gras ω-6, en particulier de la famille des acides linoléiques.

8. Préparation selon l'une des revendications 1 à 7, **caractérisée en ce que** l'acide gras insaturé est choisi dans le groupe constitué de l'acide eicosapentaénoïque, l'acide 5,8,11,14-eicosatétraénoïque et l'acide docosahexaénoïque.

9. Préparation selon l'une des revendications 1 à 8, **caractérisée en ce qu'**elle contient au moins un anti-oxydant choisi en particulier dans le groupe des vitamines hydro- et/ou lipo-solubles.

10. Préparation selon la revendication 9, **caractérisée en ce que** la vitamine liposoluble est choisie dans le groupe constitué des tocophérols, des carotinoïdes, des tocotriénols, des polyphénols, des flavonoïdes, des flavonols et des flavones, de préférence en une quantité de 4 mg à 25 mg par forme galénique en apport quotidien unique.

11. Préparation selon la revendication 10, **caractérisée en ce que** les tocophérols sont choisis dans le groupe des vitamines E, de préférence en une quantité allant jusqu'à 25 mg par forme galénique en apport quotidien unique.

12. Préparation selon la revendication 11, **caractérisée en ce que** la vitamine hydrosoluble contient de l'acide ascorbique, de préférence en une quantité de 20 mg à 100 mg par forme galénique en apport quotidien unique.

13. Préparation selon l'une des revendications 1 à 12, **caractérisée en ce qu'**elle contient de l'acide folique, de préférence en une quantité de 0,1 g à 0,6 g à une pureté de 87,8 % d'acide folique par forme galénique en apport quotidien unique et de manière encore davantage préférée, en une quantité de 0,2 à 0,5 g et de manière encore plus préférée, de 0,25 à 0,4 g.

14. Utilisation d'une préparation selon l'une des revendications 1 à 13, pour la production d'un médicament pour prévenir et/ou traiter les carences, en particulier pendant la grossesse et l'allaitement.

15. Capsule, en particulier capsule de gélatine souple, contenant une préparation selon l'une des revendications 1 à 13.
